# EUROPEAN PATENT APPLICATION

(11) **EP 3 412 254 A1**
(43) Date of publication of application: **12.12.2018**
(21) Application number: 17747038.2
(22) Date of filing: 01.02.2017
(51) Int. Cl.: A61F 5/055

(54) **CORRECTOR FOR DOUBLE CHIN AND CERVICAL VERTEBRAE**

(30) Priority: 03.02.2016 ES 201600076 U
(71) Applicant: Laboratorios Starga, S.L., 30011 Murcia (ES)
(72) Inventor: IZQUIERDO GADEA, Maria Ángeles, 30011 Murcia (ES)
(74) Representative: Monzón de la Flor, Luis Miguel
(86) International application number: PCT/ES2017/000011
(87) International publication number: WO 2017/134319

(57) **Abstract**

Corrector for double chin and cervical vertebrae, comprising a hoop (1) that is placed around the patient's neck and a positionable articulated device formed by a bridge (3) and a shaft (4) with a stop element (5) therebetween to prevent same from rotating inwards towards the neck, and a extendable telescopic arm (7) supporting an element (9) used to support the lower jaw. The moving elements are secured using screws.

## Description

### OBJECT OF THE INVENTION

The invention which is the subject of the present application and specifications consists, as the title thereof indicates, of a "double chin and cervical vertebrae".

The state of the art and the scope of industrial application lie in the world of external prosthetic devices for use as a preventive brace whose application may be performed by the user himself/herself; said use being either voluntary or by medical prescription.

### BACKGROUND OF THE INVENTION

In accordance with the analysis of the state of the art, no product featuring identical or similar characteristics exists.

Therefore, the object of the invention proposed provides fundamental advantages in its use, which are not covered by other similar or alternative means.

The "double chin and cervical vertebrae" is a device comprising an easily-fitted and easily-removed collar featuring an articulated element of variable dimensions, and with a supporting element to be placed below the head, and upon which the double chin may be positioned for its bracing and to prevent its sagging.

The device can thus maintain the head in a raised position, correcting the position of the cervical vertebrae and preventing the deformation thereof.

The habit - for reasons of comfort - of lowering the head in many situations, particularly when resting, reading, watching television, etc, causes us to adopt a poor posture, and this causes headaches, muscle pain and aching bones.

It therefore improves our aesthetic appearance, due to both the position of the head and the absence of a double chin, caused by the strengthening of the muscles at the lower part of the jaw and neck.

There are currently no similar products, there only being tubular devices, which encircle the entire neck, these being curative and not preventive; however, these do not act precisely on the region of the double chin.

As it is known by any inventor skilled in the art, the object of the invention represents a significant novelty as, due to the characteristics and advantages it presents, it is clearly of industrial and commercial interest.

### DESCRIPTION OF THE INVENTION

The "double chin and cervical vertebrae", in the form of an open hoop made preferably from metal or plastic, is circular, with a diameter approximately equal to that of the neck; the two extremities thereof at the open part of the hoop are protected by small spheres.

By flexing the material, it can be opened further, and the neck may be inserted therein, the opening remaining at the nape of the neck, and at the forward part of the neck, a bridge element with a perpendicular hollow shaft attached thereto; said shaft may swing on the axis of the bridge, and it may be locked by means of a small screw.

Another arm slides telescopically within the hollow shaft, at the extremity thereof there being a transversal element in the shape of a half-tube for the resting of the double chin thereon.

To establish the length of the telescopic shaft, the hollow shaft features a small screw for the locking of the telescopic element.

Furthermore, it is foreseen that the invention should be comfortable and easy to use, and its scrapping and elimination should likewise be convenient and easy, without endangering the environment.

As a supplement to the description to be made hereunder, and for the purpose of aiding a better understanding of the characteristics of the invention, the present specification is accompanied by a sheet of drawings, on the basis of which the innovations and advantages of the device which is the object of the invention will be understood more easily.

### BRIEF DESCRIPTION OF THE DRAWINGS

In order to understand the scope of the characteristics and advantages of the object of the invention, said object, application and specification are accompanied by a sheet bearing two drawings to complement the description of a preferred embodiment to be described hereunder; the content thereof being merely descriptive and in no way limitative.
Figure 1 portrays the object of the invention "double chin and cervical vertebrae" assembled and ready to use.
Figure 2 portrays, in profile, the silhouette of a dummy with the device in place, showing the angle of swinging of the mast which enables the placement or release thereof.

### FIGURE 1

(1) Hoop
(2) Sphere
(3) Bridge
(4) Mast
(5) Stop
(6) Mast locking screw
(7) Arm
(8) Arm locking screw
(9) Support

### FIGURE 2

(10) Angle of release

### DESCRIPTION OF A PREFERRED EMBODIMENT

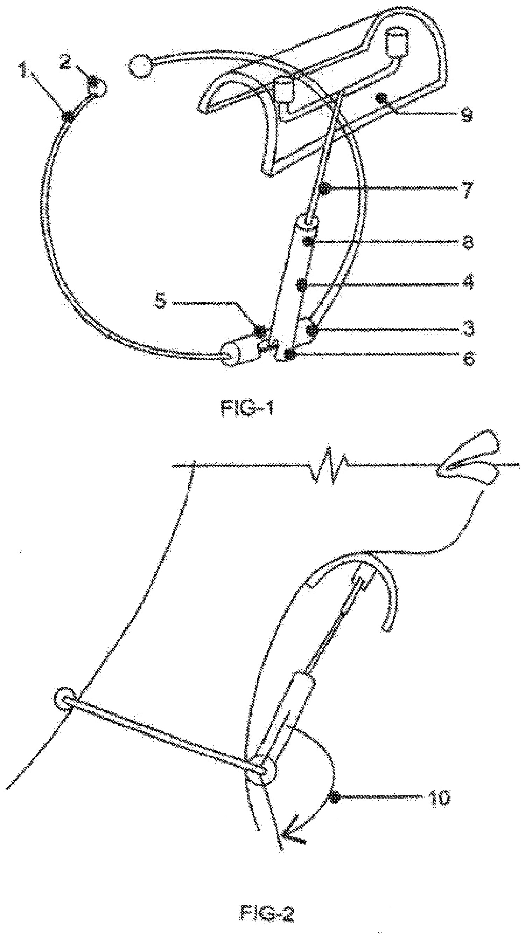

Figure 1.- The circular hoop (1), open at the section positioned at the nape of the neck, is made of a solid, flexible material with memory properties. Both extremities thereof are protected by spheres (2).

At the side opposite to the opening in the hoop, there is a widened section with a straight profile by way of a bridge (3) axis; a hollow shaft or mast (4) being affixed thereto, the swinging movement thereof being limited by a stop (5); said mast being locked on the bridge (3) by means of a mast-locking screw (6).

Within said hollow mast (4), a shaft or arm (7) may slide, this being lockable to the mast (4) by means of an arm-locking screw (8) and featuring at its extremity a supporting element (9) for the double chin.

Figure 2.- The entire device, from the mast (4) onward, can swing around the bridge (3), describing an angle of release (10) whose opening and closing depend on the placement of the support (9), and may also act as a safety device in the event of any incident related to the position of the neck.

## Claims

1. A double chin and cervical vertebrae, **characterised in that** it comprises an open hoop (1) made from a solid, flexible material, the extremities thereof being protected by two spheres (2), one at each extremity of the hoop (1); at the side opposite to the opening in the hoop, there being a widened section with a bridge (3) or straight axis on which a mast (4) is perpendicularly affixed to the bridge (3), which features a stop (5) to halt the swinging movement of the mast (4); said mast being locked on the bridge (3) and housing an arm (7) which may slide by way of a telescopic shaft and which is locked by means of an arm-locking screw (8), there being at the extremity thereof a support (9) for the double chin.

2. A double chin and cervical vertebrae in accordance with the preceding claim, **characterised in that** it features a bridge (3) on which a mast (4) is affixed and may swing, and is positioned thereon by means of a mast-locking screw (6), describing an angle of release (10), said angle being more or less closed in accordance with the user's requirements, and in the event of occasionally lowering the head, enables escape from the device.
